# EUROPEAN PATENT SPECIFICATION

(11) **EP 2 331 629 B1**
(45) Date of publication and mention of the grant of the patent: **22.06.2016**
(21) Application number: 09812572.7
(22) Date of filing: 15.09.2009
(51) Int. Cl.: C08L 3/02, A61K 47/36, A61K 9/14, C08J 3/12, C08J 3/20, C12N 11/10, C12N 1/20

(54) **STARCH-BASED MICROPARTICLES FOR THE RELEASE OF AGENTS DISPOSED THEREIN**
AUF STÄRKE BASIERENDE MIKROPARTIKEL ZUR FREISETZUNG VON DARIN ENTHALTENEN WIRKSTOFFEN
MICROPARTICULES À BASE D'AMIDON POUR LA LIBÉRATION D'AGENTS DISPOSÉS DANS CELLES-CI

(30) Priority: 15.09.2008 US 97160 P
(43) Date of publication of application: 15.06.2011
(73) Proprietor: Paladin Labs Inc., Montreal, QC H4P 2T4 (CA); Paladin Labs Europe Limited, Dublin 3 (IE); Paladin Labs (Barbados) Inc., Christ Church (BB)
(72) Inventor: RAHMOUNI, Miloud, Montréal Québec H9J 3P9 (CA); KHALID, Mohamed, Nabil, Montréal, Québec H2L 5B9 (CA); SANT, Vinayak, Montréal Québec H3N 2B9 (CA); TAFER, Abdelaziz, Lachenale Québec J6W 6K5 (CA); SMITH, Damon, Saint-Laurent Québec H4R 3A5 (CA)
(74) Representative: V.O.
(86) International application number: PCT/CA2009/001260
(87) International publication number: WO 2010/028489

(56) References cited:
- WO-A1-02/02084
- WO-A1-2009/076764
- WO-A2-2004/043140
- WO-A2-2004/091307
- US-A1- 2007 224 129

## Description

### TECHNICAL FIELD

The invention relates generally to microparticles with a high loading capacity for the stabilization and/or controlled release of one ot more agents disposed therein, and more particularly, the invention relates to starch-based microparticles with a high loading capacity for the stabilization and/or controlled release of one or more agents disposed therein.

### BACKGROUND ART

Over the years, there has been considerable interest in developing compositions that mask the taste and/or odor, stabilize the integrity, and control the release, of one or more agents disposed therein. By masking the taste and/or odor of an agent, such compositions may improve or even make possible the ability of a subject to ingest certain agents, This can have the additional benefit of patient compliance. By stabilizing the agents, the compositions can increase the usable shelf-life of the agent disposed therein. The controlled-release properties of the compositions may reduce the need for frequent administration of the agent while also improving the enjoyment, benefit, efficacy, palatability, tolerability and/or safety of the agent. For example, when the agent is a pharmaceutical, the controlled release properties can result in improved efficacy and safety for the patient by maintaining in vivo drug levels within a therapeutic range, which may not occur when the patient forgets to take or otherwise misses taking one or more doses of an immediate release dosage form.

A variety of controlled release systems have been developed to date. However, there is still an ongoing need for compositions with high loading capacity that mask taste

WO 2004/043140 refers to a method of producing a particle or microparticle comprising digestible and non-digestible polymer, a protein and a bioactive agent, but does not indicate cross-linked high amylose starch as a specific compound of the (micro)-particle.

WO 2004/091307 focus on stable, bioactive feed additive, and a method for producing same, which are not characterized by controlled release and do not comprise cross-linked high amylose starch.

The starch of WO 2002/02084 comprise cross-linked high amylose starch, which is produced by (1) granular cross-linking and additionally chemical modifications of high amylose starch, (2) thermal gelatinization of the starch from step (1), and (3) drying the starch from step (2) to yield a powder capable of being used as a controlled release excipient.

US 2007/224129 relates to coated solid microparticulate oral pharmaceutical forms, which avoid misuse of the active agent of the pharmaceutical form, wherein the coating layer imparts crushing resistance. and/or odor while maintaining or enhancing the stability of, and/or permit the controlled release of, agents disposed therein but yet are cost effective and easy to manufacture.

### DISCLOSURE OF INVENTION

In one aspect, the invention provides a method of producing microparticles that have a high loading capacity while enhancing the stability and/or control the release of one or more agents, for example, a pharmaceutical, a flavoring agent, a nutraceutical, an agricultural agent, a cosmetic agent, or a combination thereof, disposed therein. The resulting microparticles, either inherently or by the inclusion of specific components, can also mask the taste and/or odor of one or more of the agents disposed therein.

The method comprises two steps. The first step comprises providing a solution comprising a mixture of from about S % (w/w) to about 20 % (w/w) of cross-linked high amylose starch (for example, from about 7 % (w/w) to about 15 % (w/w) of cross-linked high amylose starch), and the agent to be released. The second step comprises spray drying the mixture in a spray dryer to produce microparticles having a mean diameter of from about 1 µm to about 200 µm. The agent or agents may be disposed within the lumen of the microparticles and/or the wall of the microparticles. The spray dryer employed preferably has an air inlet temperature in the range of from about 125°C to about 250°C and an air outlet temperature in the range of from about 50°C to about 100°C, or from about 70°C to about 100°C. The agent can comprise from about 5 % to about 50 % (w/w), from about 10 % to about 45 % (w/w), from about 15 % to about 40 % (w/w), from about 15 % to about 35 % (w/w), or from about 20 % to about 35 % (w/w) of the resulting microparticles. It is understood that the microparticles can be hollow.

In another aspect, the invention provides a microparticle composition comprising a plurality of microparticles comprising from about 35 % (w/w) to about 70 % (w/w) of cross-linked high amylose starch and one or more agents for release from the microparticles. The microparticles have a mean diameter in the range from about 1 µm to about 200 µm. The agent or agents can be disposed within the lumen or the microparticles and/or within the wall or walls of the microparticles. In certain embodiments, the agent comprises from about S % to about 50 % (w/w), from about 10 % to about 45 % (w/w), from about 15 % to about 40 % (w/w), from about 15 % to about 35 % (w/w), oi from about 20 % to about 35 % (w/w) of the resulting microparticlea. In ocrtain other embodiments, the microparticles are substantially free of pectin and/or are substantially resistant to degradation by α-amylase.

These and other aspects and features of the invention are described in the following detailed description and claims.

### BRIEF DESCRIPTION OF THE DRAWINGS

The invention is illustrated but is not limited by the annexed drawings, in which
FIGURE 1 is a schematic representation of an exemplary procedure for producing the microparticles of the invention;
FIGURE 2 is an image taken by a scanning election microscope of spray dried CONTRAMID® excipient-based microparticles containing 16.5 % (w/w) tramadol HCl;
FIGURE 3 is an image taken by a scanning electron microscope of a cross- section of a spray dried CONTRAMID® excipient -based microparticle;
FIGURE 4 is a graph showing the in vitro release profiles of agents from exemplary microparticles loaded with either 16.5% (w/w) tramadol HCl (-•-) or caffeine (-■-);
FIGURE 5 is a graph showing the in vitro release profiles of tramadol HCl from microparticles in water (-•-) or water containing α-amylase (-Δ-);
FIGURE 6 is an image taken by a scanning electron microscope of spray dried CONTRAMID® excipient-based microspheres containing about 50% (w/w) tramadol HCl;
FIGURE 7 is a graph showing the in vitro release profile of tramadol HCl from exemplary microparticles containing about 50% (w/w) tramadol HCl (-•-);
FIGURE 8 is an image taken by a scanning electron microscope of the bacterial strain *Lacobacillus rhamnosus;* and
FIGURE 9 is an image taken by a scanning electron microscope of spray dried CONTRAMID® excipient-based microspheres containing the bacterial strain *Lacobacillus rhamnosus.*

### BEST MODES OF CARRYING OUT THE INVENTION

The invention is based, in part, upon the discovery that it is possible to produce starch-based microparticles with a high loading capacity that provide taste and/or odor masking properties while enhancing the stability and/or permitting the controlled release of one or more agents, for example, a pharmaceutical, a flavoring agent, a taste masking agent, a nutraceutical, a health supplement, a probiotic, an agricultural agent, a cosmetic agent, or a combination thereof, disposed within the microparticles.

### I. MICROPARTICLES AND THEIR MANUFACTURE

The starch-based microparticles are produced by a two step procedure. The first step comprises providing a solution comprising a relatively high concentration of cross- linked high amylose starch (for example, from about 5 % (w/w) to about 20 % (w/w) of cross-linked high amylose starch, and more particularly, from about 7 % (w/w) to about 15% (w/w) of cross-linked high amylose starch) and the agent or agents to be released. The second step comprises spray drying the mixture in a spray dryer to produce microparticles having a mean diameter of from about 1 µm to about 200 µm, from about 5 µm to about 200 µm, from about 5 µm to about 150 µm, from about µm to about 100 µm, from about 10 µm to about 100 µm, from about 1 µm to about 50 µm, from about 1 µm to about 40 µm, from about µm to about 30 µm, from about 2 µm to about 50 µm, from about 3 µm to about 50 µm, from about 4 µm to about 50 µm, or from about 5 µm to about 50 µm. The spray dryer employed preferably has an air inlet temperature in the range of from about 125°C to about 250°C, and an air outlet temperature in the range of from about 50°C to about 100°C, or from about 70°C to about 100°C.

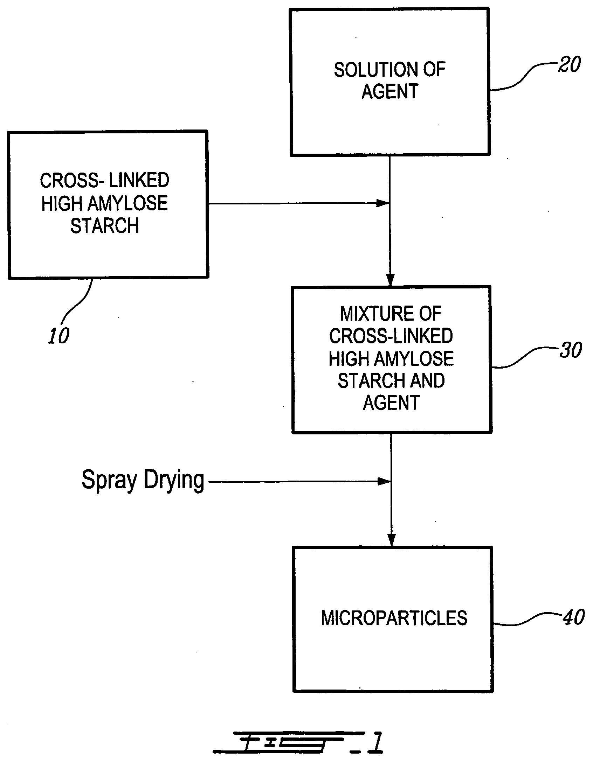

**FIGURE 1** is a schematic representation of an exemplary protocol for producing the microparticles of the invention. A solution comprising from about 5 % (w/w) to about 20% (w/w), or from about 7 % (w/w) to about IS % (w/w) of cross-linked high amylose starch 10 is admixed with a solution 20 comprising one or more agents of interest and an optional viscosity reducing agent and an optional dispersing agent, to produce mixture 30 comprising the cross-linked high amylose starch, the agent(s) of interest, and the optional viscosity reducing agent and the optional dispersing agent. The mixture 30 generally is created at a temperature in the range of from about 15°C to about 70°C, or in the range of from about 30°C to about 60°C. Mixture 30 then is spray dried to produce a preparation of microparticles 40. Methods for performing spray drying are described, for example, in Giunchedi and Conte (1995) S.T.P. PHARMA SCIENCES 5:276-290; Wendel &. Celik (1997) PHARMACEUTICAL TECHNOLOGY 124-156. It is understood, however, that the protocol can be modified, for example, as discussed in more detail below, when the agent of interest is sparingly soluble, slightly soluble, or insoluble in an aqueous solvent, for example, water, an aqueous buffer, water-alcohol mixtures, or aqueous buffer-alcohol mixtures. It is understood that the microparticles can be hollow thereby resulting in hollow microparticles.

When the agent or agents to be included in the microspheres include agents that are poorly soluble or insoluble in water (for example, menthol (see, Example 3) and vanillin (see, Example 4), the agents can be heated to form a dispersion and then are combined with cross-linked high amylose starch 10 to produce a mixture 30 that is an emulsion. The emulsion can then be spray dried to produce a preparation of microparticles 40.

A variety of cross-linked high amylose starches may be used in the practice of the invention. The cross-linking of high amylose starch can be produced using procedures described in the art. For example, cross-linking of amylose can be carried out in the manner described in Mateescu [BIOCHEMIB 60: 535-537 (1978)] by reacting amylose with epichlorohydrin in an alkaline medium. In the same manner, starch can also be cross-linked with a reagent selected from the group consisting of epichlorohydrin, adipic acid anhydride, sodium trimetaphosphate and phosphorous oxychloride or other cross- linking agents including, but not limited to, 2,3-dibromopiOpanol, linear mixed anhydrides of acetic and di- or tribasic carboxylic acids, vinyl sulfone, diepoxides, cyanuric chloride, hexahydro-1,3,5-trisacryloyl-s-triazine, hexamethylene diisocyanate, toluene 2,4-düsocyanate, N,N-methylenebisacrylatnide, N,N'-bis (hydroxymethyl) ethyleneurea, mixed carbonic-carboxylic acid anhydrides, imidazolides of carbonic and polybasic carboxylic acids, imidazolium salts of polybasic carboxylic acids, and guanidine derivatives of polycarboxylic acids. The reaction conditions employed will vary with the type and amount of the cross-linking agent that is used, as well as the base concentration, amount and type of starch. In some embodiments, the cross-linked high amylose starch may be gelatinized after cross-linking,

It is contemplated that starches containing more than about 40% w/w amylose can be used to form cross-linked high amylose starch, e.g., pea and wrinkled pea starch, bean starch, hybrids or genetically modified tapioca or potato starch, or any other root, tuber or cereal starch. Preferably, high amylose starch containing about 70% w/w amylose is used as the base material. For example, high amylose starch, Cerestar AmyloGel 03003 (Cerestar U.S.A. Inc.) can be used. In certain formulations, the excipient comprises cross-linked high amylose starch comprising between about 65% and about 75% by weight of amylose cross-linked with phosphorus oxychloride.

In one embodiment, the cross-linked high amylose starch is cross-linked with phosphorus oxychloride and/or comprises hydroxypropyl side chains. Exemplary cross- linked high amylose starch has been developed by and is available commercially from Labopharm, Inc., Laval, Canada, under the tradename CONTRAMID®. The synthesis of the CONTRAMID® excipient is described, for example, in U.S. Patent No. 6,607,748.

In certain embodiments, when the agent is mixed with the cross-linked high amylose starch, a high shear mixer can be used to reduce the viscosity of the resulting mixture. The high shear mixer can reduce the mixture to uniform-sized molecules through maceration, cutting and blending, thus reducing the thickness as well as the viscosity of the solution. Suitable high shear mixers include, for example, the MF550 Turbo from Robot® Coupe (Jackson, MS) and the IKA Ultra-Turrax T-2S Basic S1 from IKA Works (Wilminton, NC) and are used in accordance with the manufacturer's instructions.

Alternatively, the appropriate viscosity of the mixture can be achieved by mixing the cross-linked high amylose starch with a viscosity reducing agent, either with or without high shear mixing. The viscosity reducing agent can be selected from the group consisting of a water soluble linear polymer, a water soluble linear copolymer, and a polyol, In certain embodiments, the viscosity reducing agent is selected from the group consisting of a polyvinylpyrrolidone-vinyl acetate copolymer, polyvinyl pyrrolidone, propylene glycol, maltodextrin, sorbitol, and mannitol. In a preferred embodiment, the viscosity reducing agent is polyvinylpyrrolidone-vinyl acetate copolymer, also sold under the trade name KOLLIDON VA 64 Fine from BASF, New Jersey.

In certain embodiments, in the liquid mixture prior to or in the resulting composition after spray drying, the ratio of the cross-linked high amylose starch to the optional viscosity reducing agent is from about 80:20 (w/w) to about 40:60 (w/w), for example, 80:20 (w/w), 75:25 (w/w), 70:30 (w/w), 65:35 (w/w), 60:40 (w/w). 55:45 (w/w), 50:50 (w/w), 45:55 (w/w), or 40:60 (w/w). In certain embodiments, the ratio of the cross-linked high amylose starch to the optional viscosity reducing agent is about 60:40 (w/w). In certain embodiments, the mixture prior to or after spray drying is substantially free of pectin, Le., has less than about 3 % (w/w), less than about 2 % (w/w) or less than about 1 % (w/w) pectin.

In certain embodiments, the agent is soluble in an aqueous solvent (e.g., 1 gram of agent dissolves in less than 1 mL or up to about 30 mL of aqueous solvent), or the agent is sparingly soluble in an aqueous solvent (e.g., 1 gram of agent dissolves in greater than 30 mL to about 100 mL of aqueous solvent), or the agent is slightly soluble in aqueous solvent (e.g., 1 gram of agent dissolves in from about 100 mL to about 10,000 mL of aqueous solvent), or the agent is insoluble in an aqueous solvent (e.g., 1 gram of agent dissolves in greater than 10,000 mL of aqueous solvent). The method, however, can be modified as described below depending upon the melting point of the agent. For example, if the agent has a low melting point (e.g., below 60°C), the agent can be melted by heating and then added to the cross-linked high amylose starch to form an emulsion. Alternatively, if the agent has a high melting point (e.g., above 60°C), the agent can be combined with a dispersing agent and then added to the cross-linked high amylose starch to form an oily emulsion. In either approach, an optional surface active agent, such as Tween® (for example, Tween®20 or Tween® 80), can be added to the emulsion in order to improve the stability of the emulsion.

Exemplary dispersing agents include, for example, oils, for example, mineral oil and vegetable oils, including for example canola oil, corn oil, cottonseed oil, coconut oil, cocoa butter, grapeseed oil, hemp oil, olive oil, palm oil, peanut oil, rapeseed oil, safflower oil, sesame oil, soybean oil, sunflower oil and the like. Other exemplary dispersing agents include surfactants and emulsifiers, for example, alginates, carageenan, xanthan and other carbohydrate gums, modified starches, cellulose derivatives, fatty acids and their salts, glycerides and esters thereof, sorbitan esters, polysorbates (Tween®, for example, Tween® 20 or Tween® 80), polyoxyethylene stearates, poloxamers, sodium dodecyl sulphate, and the like.

The spray dried microparticle compositions described herein comprise a plurality of microparticles comprising from about 35 % (w/w) to about 70 % (w/w) of cross-linked high amylose starch and an agent for release from the microparticles, wherein the microparticles have a mean diameter in the range from about 1µm to about 200 µm.. Depending upon the circumstances, it can be desirable to include an optional viscosity reducing agent and/or dispersing agent in the microparticles. The microparticles, in certain embodiments, are substantially free of pectin and/or are substantially resistant to degradation by α-amylase (for example, the release of the agent from the microparticles in water containing 5000 Units/L of α- amylase is within 20%, more preferably within 10% of the release of the agent in water in the absence of α-amylase).

The microparticles described herein can be disposed within a capsule, tablet, caplet, chewing gum, film, wafer or an orally disintegrating tablet, Such formulations can be orally administered to an individual in need of such an agent.

### II AGENTS INCORPORATED INTO THE MICROPARTICLES

It is understood that the agent incorporated into the microparticles can be a pharmaceutical, a taste masking agent, a flavoring agent, a nutraceutical (for example, a botanical or herbal extract, a health supplement, or a weight loss agent), a health supplement, a probiotic agent, a cosmetic agent, an agricultural agent, or a combination thereof.

For example, it is contemplated that the microparticles can contain (i) a pharmaceutical, (ii) a pharmaceutical and a taste masking agent, (iii) a pharmaceutical and a flavoring agent, and (iv) a pharmaceutical, a taste masking a agent and a flavoring agent. Furthermore, it is contemplated that the microparticles can comprise a plurality of different pharmaceuticals and optionally a taste masking agent and/or a flavoring agent. It is understood that in certain embodiments, based on the fact that the microparticles have controlled release properties, the microparticles inherently have taste masking properties because the pharmaceutical is released in the mouth at a rate so that any taste is reduced or eliminated. Furthermore, it is understood that in each of the foregoing formulations, the pharmaceutical can be replaced by or supplemented with one or more of a nutraceutical, a health supplement, a probiotic agent, a cosmetic agent or an agricultural agent.

The microparticles produced by the methods described herein have a high loading capacity for the agent. In certain embodiments, the agent or agents incorporated into the microparticles comprise from about 5 % to about 50 % (w/w) of the microparticles, for example, from about 10 % to about 50 % (w/w), from about 20 % to about 50 % (w/w), from about 30 % to about 50 % (w/w), from about 40 % to about 50% (w/w), from about 5 % to about 40 % (w/w), from about S % to about 30 % (w/w), from about 5 % to about 20 % (w/w), from about 10 % to about 45 % (w/w), from about 10 % to about 40 % (w/w), from about 10 % to about 30 % (w/w), from about 10 % to about 20 % (w/w), from about 15 % to about 40 % (w/w), from about IS % to about 35 % (w/w), from about 15 % to about 30 % (w/w), from about 20 % lo about 35 % (w/w), or from about 20 % to about 30 % (w/w) of the microparticles.

The loading capacity of the microparticles can be determined using a number of protocols known in the art. In one exemplary approach, a fraction of the microparticles of interest are weighed and transferred to a 100 mL volumetric flask. Then, depending upon the physical properties of the agent, the agent is extracted using an appropriate solvent. For example, for a water soluble agent, for example, tramadol, 60 mL of a water-acetonitrite mixture (40:60 (v/v)) is added to the flask. Following shaking for 1-5 minutes and then sonication using a Branson 8510 (Danbury, CT) sonic bath for 10 to about 30 minutes, the solution is permitted to equilibrate for 10 to about 30 minutes. The solution then is made up to 100 mL with the water-acetonitrite mixture. Then 5 mL of the resulting solution is diluted 20-fold with water-acetonitrite (77:23 (v/v)). After filtration to remove particles (for example, via filtering through a 0.4S µm PTFE filter), the diluent is analyzed by high pressure liquid chromatography (HPLC). Based on the results from the HPLC analysis, it is possible to determine the amount (percent w/w) of agent in the fraction of microparticles analyzed. It is understood, however, that the actual protocol and reagents (e.g., solvents) may vary depending upon what agent or agents are incorporated into the microparticles.

### A - PHARMACEUTICALS

The microparticles described herein are particularly useful in the delivery of pharmaceuticals.

The terms "pharmaceutical," "pharmaceutically active agent," and "active pharmaceutical ingredient" are used interchangeably herein, and refer to any chemical moiety that is a biologically, physiologically, or pharmacologically active substance that acts locally or systemically in a subject, and includes pharmaceutically acceptable salts, esters and prodrugs thereof. It is understood that the pharmaceutically active agent can be a small molecule, synthetic molecule, or biologic, for example, a protein, peptide, glycoprotein, or nucleic acid. Exemplary pharmaceuticals are described in well-known literature references such as the Merck Index, the Physicians Desk Reference, and The Pharmacological Basis of Therapeutics, and include, without limitation, medicaments; vitamins; mineral supplements; substances used for the treatment, prevention, diagnosis, cure or mitigation of a disease or illness; substances which affect the structure or function of the body; or pro-drugs, which become biologically active or more active after they have been placed in a physiological environment

Compositions and formulations contemplated herein may include one or more pharmaceuticals. For example, a composition may include two, three or more different pharmaceuticals.

The pharmaceuticals can vary widely with the purpose for the composition. It is contemplated that one or a plurality of different pharmaceuticals are included in the microparticles described herein. Non-limiting examples of broad categories of useful pharmaceuticals include the following therapeutic categories: anabolic agents, .antacids, anti-asthmatic agents, anti-cholesterolemic and anti-lipid agents, anti-coagulants, anticonvulsants, anti-diarrhcals, anti-emetics, anti-infective agents, antiinflammatory agents, anti-manic agents, anti-nauseants, antineoplastic agents, anti-obesity agents, anti-pyretic and analgesic agents, anti-spasmodic agents, anti-thrombotic agents, anti-uricemic agents, anti-anginal agents, antihistamines, anti-tussives, appetite suppressants, biologicals, cerebral dilators, coronary dilators, decongestants, diuretics, diagnostic agents, erythropoietic agents, expectorants, gastrointestinal sedatives, hyperglycemic agents, hypnotics, hypoglycemic agents, ion exchange resins, laxatives, mineral supplements, mucolytic agents, neuromuscular drugs, peripheral vasodilators, psychotropics, sedatives, stimulants, thyroid and antithyroid agents, uterine relaxants, vitamins, and pro-drugs.

More specifically, non-limiting examples of useful pharmaceutically active substances include the following therapeutic categories: analgesics, such as, nonsteroidal anti-inflammatory drugs, opiate agonists and salicylates; antihistamines, such as, H₁- blockers and H₂-blockers; anti-infective agents, such as, anthelmintics, antianaerobics, antibiotics, aminoglycoside antibiotics, antifungal antibiotics, cephalosporin antibiotics, macrolide antibiotics, miscellaneous β-lactam antibiotics, penicillin antibiotics, quinolone antibiotics, sulfonamide antibiotics, tetracycline antibiotics, antimycobacterials, antituberculosis antimycobacterials, antiprotozoals, antimalarial antiprotozoals, antiviral agents, antiretroviral agents, scabicides, and urinary anti-infectives; antineoplastic agents, such as alkylating agents, nitrogen mustard alkylating agents, nitrosourea alkylating agents, antimetabolites, purine analog antimetabolites, pyrimidine analog antimetabolites, hormonal antineoplastics, natural antineoplastics, antibiotic natural antineoplastics, and vinca alkaloid natural antineoplastics; autonomic agents, such as, anticholinergics, aαtimuscarinic anticholinergics, ergot alkaloids, parasympathomimetics, cholinergic agonist parasympathomimetics, cholinesterase inhibitor parasympathomimetics, sympatholytics, α-blocker sympatholytics, β-blocker sympatholytics, sympathomimetics, and adrenergic agonist sympathomimetics; cardiovascular agents, such as, antianginals, β-blocker antianginals, calcium-channel blocker antianginals, nitrate antianginals, antiarrhythmics, cardiac glycoside antiarrhythmics, class I antiarrhythmics, class II antiarrhythmics, class III antiarrhythmics, class IV antiarrhythmics, antihypertensive agents, α-blocker antihypertensives, angiotensin-converting enzyme inhibitor (ACE inhibitor) antihypertensives, β-blocker antihypertensives, calcium-channel blocker antihypertensives, central-acting adrenergic antihypertensives, diuretic antihypertensive agents, peripheral vasodilator antihypertensives, antilipemics, bile acid sequestrant antilipemics, HMG-COA reductase inhibitors, inotropes, cardiac glycoside inotropes, and thrombolytic agents; dermatological agents, such as, antihistamines, anti-inflammatory agents, corticosteroid anti-inflammatory agents; electrolytic and renal agents, such as, acidifying agents, alkalinizing agents, diuretics, carbonic anhydrase inhibitor diuretics, loop diuretics, osmotic diuretics, potassium-sparing diuretics, thiazide diuretics, electrolyte replacements, and uricosuric agents; enzymes, such as, pancreatic enzymes and thrombolytic enzymes; gastrointestinal agents, such as, antidiarrheals, antiemetics, gastrointestinal anti-inflammatory agents, salicylate gastrointestinal anti-inflammatory agents, antacid anti-ulcer agents, gastric acid-pump inhibitor anti-ulcer agents, gastric mucosal anti-ulcer agents, H2 -blocker anti-ulcer agents, cholelitholytic agents, digestants, emetics, laxatives and stool softeners, and prokinetic agents; hematological agents, such as, antianemia agents, hematopoietic antianemia agents, coagulation agents, anticoagulants, hemostatic coagulation agents, platelet inhibitor coagulation agents, thrombolytic enzyme coagulation agents, and plasma volume expanders; hormones and hormone modifiers, such as, abortifacients, adrenal agents, corticosteroid adrenal agents, androgens, anti-androgens, antidiabetic agents, sulfonylurea antidiabetic agents, antihypoglycemic agents, oral contraceptives, progestin contraceptives, estrogens, fertility agents, oxytocics, parathyroid agents, pituitary hormones, progestins, antithyroid agents, thyroid hormones, and tocolytics; immunobiologic agents, such as, immunoglobulins, immunosuppressives, toxoids, and vaccines; local anesthetics, such as, amide local anesthetics and ester local anesthetics; musculoskeletal agents, such as, anti- gout anti-inflammatory agents, corticosteroid anti-inflammatory agents, gold compound anti-inflammatory agents, immunosuppressive anti-inflammatory agents, nonsteroidal anti-inflammatory drugs (NSAIDs), salicylate anti-inflammatory agents, skeletal muscle relaxants, neuromuscular blocker skeletal muscle relaxants, and reverse neuromuscular blocker skeletal muscle relaxants; neurological agents, such as, anticonvulsants, barbiturate anticonvulsants, benzodiazepine anticonvulsants, anti-migraine agents, anti-parkinsonian agents, anti-vertigo agents, opiate agonists, and opiate antagonists; psychotropic agents, such as, antidepressants, heterocyclic antidepressants, monoamine oxidase inhibitors

(MAOIs), selective serotonin re-uptake inhibitors (SSRIs), tricyclic antidepressants, antimanics, antipsychotics, phenothiazine antipsychotics, anxiolytics, sedatives, and hypnotics, barbiturate sedatives and hypnotics, benzodiazepine anxiolytics, sedatives, and hypnotics, and psychostimulants; respiratory agents, such as, antitussives, bronchodilators, adrenergic agonist bronchodilators, antimuscarinic bronchodilators, expectorants, mucolytic agents, respiratory anti-inflammatory agents, and respiratory corticosteroid anti-inflammatory agents; toxicology agents, such as, antidotes, heavy metal antagonists/chelating agents, substance abuse agents, deterrent substance abuse agents, and withdrawal substance abuse agents; minerals; and vitamins, such as, vitamin A, vitamin B, vitamin C, vitamin D, vitamin E, and vitamin K.

Preferred classes of useful pharmaceuticals from the above categories include: (1) nonsteroidal anti-inflammatory drugs (NSAIDs) analgesics, such as, diclofenac, ibuprofen, ketoprofen, and naproxen; (2) opiate agonist analgesics, such as, codeine, fentanyl, tramadol, hydromoiphone, hydrocodone, oxycodone, oxymorphone and morphine; (3) salicylate analgesics, such as, aspirin; (4) HI-blocker antihistamines, such as, clemastine and terfenadine; (S) H2 -blocker antihistamines, such as, cimetidine, famotidine, nizadine, and ranitidine; (6) anti-infective agents, such as, mupirocin; (7) antianaerobic anti-infectives, such as, chloramphenicol and clindamycin; (8) antifungal antibiotic anti-infectives, such as, amphotericin b, clotrimazole, fluconazole, and ketocona2θle; (9) macrolide antibiotic anti-infectives, such as, azithromycin and erythromycin; (10) miscellaneous β-lactam antibiotic anti-infectives, such as, aztreonam and imipenem; (11) penicillin antibiotic anti-infectives, such as, nafcillin, oxacillin, penicillin G, and penicillin V; (12) quinolone antibiotic anti-infectives, such as, ciprofloxacin and norfloxacin; (13) tetracycline antibiotic anti-infectives, such as, doxycycline, minocycline, and tetracycline; (14) antituberculosis antimycobacterial anti- infectives such as, isoniazid (INH), and rifampin; (15) antiprotozoal anti-infectives, such as, atovaquone and dapsone; (16) antimalarial antiprotozoal anti-infectives, such as, chloroquine and pyrimethamine; (17) anti-retroviral anti-infectives, such as, ritonavir and zidovudine; (18) antiviral anti-infective agents, such as, acyclovir, ganciclovir, interferon alfa, and rimantadine; (19) alkylating antineoplastic agents, such as, carboplatin and cisplatin; (20) nitrosourea alkylating antineoplastic agents, such as, carmustine (BCNU); (21) antimetabolite antineoplastic agents, such as, methotrexate; (22) pyrimidinc analog antimetabolite antineoplastic agents, such as, fluorouracil (5-FU) and gemcitabine; (23) hormonal antineoplastics, such as, goserelin, leuprolide, and tamoxifen; (24) natural antineoplastics, such as, aldesleukin, interleukin-2, docetaxel, etoposide (VP-16), interferon alfa, paclitaxel, and tretinoin (ATRA); (25) antibiotic natural antineoplastics, such as, bleomycin, dactinomycin, daunorubicin, doxorubicin, and mitomycin; (26) vinca alkaloid natural antineoplastics, such as, vinblastine and vincristine; (27) autonomic agents, such as, nicotine; (28) anticholinergic autonomic agents, such as, benztropine and trihexyphenidyl; (29) antimuscarinic anticholinergic autonomic agents, such as, atropine and oxybutynin; (30) ergot alkaloid autonomic agents, such as, bromocriptine; (31) cholinergic agonist parasympathomimetics, such as, pilocarpine; (32) cholinesterase inhibitor parasympathomimetics, such as, pyridostigmine; (33) α-blocker sympatholytics, such as, prazosin; (34) 9-blocker sympatholytics, such as, atenolol; (35) adrenergic agonist sympathomimetics, such as, albuterol and dobutamine; (36) cardiovascular agents, such as, aspirin; (37) i-blocker antianginals, such as, atenolol and propranolol; (38) calcium-channel blocker antianginals, such as, nifedipine and verapamil; (39) nitrate antianginals, such as, isosorbide dinitrate (ISDNT); (40) cardiac glycoside antiarrhythmics, such as, digoxin; (41) class I antiarrhythmics, such as, lidocaine, mexiletine, phenytom, procainamide, and quinidine; (42) class II antiatrhythmics, such as, atenolol, metoprolol, propranolol, and timolol; (43) class III antiarrhythmics, such as, amiodaiøne; (44) class IV antiarrhythmics, such as, diltiazem and verapamil; (45) α blocker antihypertensives, such as, prazosin; (46) angiotensin-converting enzyme inhibitor (ACE inhibitor) antihypertensives, such as, captopril and enalapril; (47) β-blocfccr antihypertensives, such as, atenolol, metoprolol, nadolol, and propanolol; (48) calcium-channel blocker antihypertensive agents, such as, diltiazem and nifedipine; (49) central-acting adrenergic antihypertensives, such as, clonidine and methyldopa; (50) diurectic antihypertensive agents, such as, amiloride, furosemide, hydrochlorothiazide (HCTZ), and spironolactone; (51) peripheral vasodilator antihypertensives, such as, hydralazine and minoxidil; (52) antilipem.es, such as, gemfibrozil and probucol; (53) bile acid sequestrant antilipemics, such as, cholestyramine; (54) HMO-CoA reductase inhibitor antilipcmics, such as, lovastatin and pravastatin; (55) inotropes, such as, amrinone, dobutamine, and dopamine; (56) cardiac glycoside inotropes, such as, digoxin; (57) thrombolytic agents, such as, alteplase (TPA), anistreplase, streptokinase, and urokinase; (58) dermatological agents, such as, colchicine, isotretinoin, methotrexate, minoxidil, tretinoin (ATRA); (59) dermatological corticosteroid anti-inflammatory agents, such as, betamethasone and dexamethasone; (60) antifungal anti-infectives, such as, amphotericin B, clotrimazole, miconazole, and nystatin; (61) antiviral anti-infectives, such as, acyclovir; (62) antineoplastics, such as, fluorouracil (5-FU); (63) electrolytic and renal agents, such as, lactulose; (64) loop diuretics, such as, furosemide; (65) potassium-sparing diuretics, such as, triamterene; (66) thiazide diuretics, such as, hydrochlorothiazide (HCTZ); (67) uricosuric agents, such as, probenecid; (68) enzymes, such as, RNase and DNase; (69) thrombolytic enzymes, such as, alteplase, anistreplase, streptokinase and urokinase; (70) antiemetics, such as, prochlorperazine; (71) salicylate gastrointestinal anti-inflammatory agents, such as, sulfasalazine; (72) gastric acid-pump inhibitor anti-ulcer agents, such as, omeprazole; (73) H2-blocker anti-ulcer agents, such as, cimetidine, famotidine, nizatidine, and ranitidine; (74) digestants, such as, pancrelipase; (75) prokinctic agents, such as, erythromycin; (76) fentanyl; (77) hematopoietic antianemia agents, such as, erythropoietin, filgrastim (G-CSF), and sargramostim (GM-CSF); (78) coagulation agents, such as, antihemophilic factors 1-10 (AHF 1-10); (79) anticoagulants, such as, warfarin; (80) thrombolytic enzyme coagulation agents, such as, alteplase, anistreplase, streptokinase and urokinase; (81) hormones and hormone modifiers, such as, bromocriptine; (82) abortifacients, such as, methotrexate; (83) antidiabetic agents, such as, insulin; (84) oral contraceptives, such as, estrogen and progestin; (85) progestin contraceptives, such as, levonorgestrel and norgcstrel; (86) estrogens, such as, conjugated estrogens, diethylstilbestrol (DES), estrogen (estradiol, estrone, and estropipate); (87) fertility agents, such as clomiphene, human chorionic gonadatropin (HCG), and menotropins; (88) parathyroid agents, such as, calcitonin; (89) pituitary hormones, such as, desmopressin, goserelin, oxytocin, and vasopressin (ADH); (90) progestins, such as, medroxyprogesterone, norethindrone, and progesterone; (91) thyroid hormones, such as, levothyroxine; (92) immunobiologic agents, such as, interferon beta-lb and interferon gamma-lb; (93) immunoglobulins, such as, immune globulin IM, IMIG, IGIM and immune globulin IV, IVIG, IGIV; (94) amide local anesthetics, such as, lidocaine; (9S) ester local anesthetics, such as benzocaine and procaine; (96) musculoskeletal corticosteroid antiinflammatory agents, such as, beclomethasone, betamethasone, cortisone, dexamethasone, hydrocortisone, and prednisone; (97) musculoskeletal antiinflammatory immunosuppressives, such as, azathioprine, cyclophosphamide, and methotrexate; (98) musculoskeletal nonsteroidal anti-inflammatory drugs (NSAIDs), such as. diclofenac, ibuprofen, ketoprofen, ketorolac, and naproxen; (99) skeletal muscle relaxants, such as, baclofen, cyclobenzaprine, and diazepam; (100) reverse neuromuscular blocker skeletal muscle relaxants, such as, pyridostigmine; (101) neurological agents, such as, nimodipine, riluzole, tacrine, trazodone, and ticlopidine; (102) anticonvulsants, such as, carbamazepine, gabapentin, lamotrigine, phenytoin, and valproic acid; (103) barbiturate anticonvulsants, such as, phenobarbital and primidone; (104) benzodiazepine anticonvulsants, such as, clonazepam, diazepam, and lorazepam; (105) anti-parkinsonian agents, such as, bromocriptine, levodopa, carbidopa, and pergolide; (106) anti-vertigo agents, such as, meclizine; (107) opiate agonists, such as, codeine, fentanyl, hydromorphone, methadone, tramadol, and morphine; (108) opiate antagonists, such as, naloxone; (109) β-blocker anti-glaucoma agents, such as, timolol; (110) miotic anti-glaucoma agents, such as, pilocarpine; (111) ophthalmic aminoglycoside antiinfectives, such as, gentamicin, neomycin, and tobramycin; (112) ophthalmic quinolone anti-infectives, such as, ciprofloxacin, norfloxacin, and ofloxacin; (113) ophthalmic corticosteroid anti-inflammatory agents, such as, dexamethasone and prednisolone; (114) ophthalmic nonsteroidal anti-inflammatory drugs (NSAIDs), such as, diclofenac; (115) antipsychotics, such as, clozapine, haloperidol, and risperidone; (116) benzodiazepine anxiolytics, sedatives and hypnotics, such as, alprazolam, clonazepam, diazepam, lorazepam, oxazepam, and prazepam; (117) psychostimulants, such as, methylphenidate and pemoline; (118) antitussives, such as, codeine; (119) bronchodilators, such as, theophylline; (120) adrenergic agonist bronchodilators, such as, albuterol; (121) respiratory corticosteroid anti-inflammatory agents, such as, dexamethasone; (122) antidotes, such as, flumazenil and naloxone; (123) heavy metal antagonists/chelating agents, such as, penicillamine; (124) deterrent substance abuse agents, such as, disulfiram, naltrexone, and nicotine; (125) withdrawal substance abuse agents, such as, bromocriptine; (126) minerals, such as, Iron, calcium, and magnesium; (127) vitamin B compounds, such as, cyanocobalamin (vitamin Bi2) and niacin (vitamin B3); (128) vitamin C compounds, such as, ascorbic acid; (129) vitamin D compounds, such as, calcitriol, and (130) histamine type drugs, such as, betahistine hydrochloride.

### B - TASTE MASKING AGENTS

The mioroparticlcs, when desired, can also contain one or more taste masking agents. Exemplary taste masking agents include, without limitation, (1) sugar alcohols, for example, isomalt, maltitol, sorbitol, xylitol, mannitol, erythritol, lactitol, and glycerol (2) sugars, for example, sucrose, glucose (com syrup), dextrose, invert sugar, fructose, and polydextiOse. (3) cellulose, (4) saccharine and its various salts, such as, the sodium salt or the calcium salt; (S) cyclainic acid and its various salts, such as, the sodium salt, (6) dipeptide sweeteners, for example, aspartame, alitame, and neotame, (7) dihydrochalone compounds, (8) glycyrrhizin, (9) stevia Rebaudiana (Stevioside), (10) thaumatin, (11) dihydroflavinol, (12) hydroxyguaiacol esters, (13) L-amino dicaboxylic acid gem-diamines, (14) L-aminodicarboxylic acid aminoalkenoic acid ester amides, and (15) synthetic sweeteners, for example, acesulfame potassium, sucralose, 3,6-dihydro-6^{~} methyl-1-1,2,3-oxathiaziii-4-one-2,2-dioxide, and salts thereof.

### C- FLAVORING AGENTS

The microparticles, when desired, can also contain one or more flavoring agents. Exemplary flavoring agents include, without limitation, (1) natural and synthetic flavoring agents, for example, mints (for example, peppermint and spearmint), menthol, artificial vanilla, vanillin, ethyl vanillin, cinnamon, cinnamyl alcohol, methyl cinnamate, ethyl cinamate, methyl benzoate, clove, chlorophyll, eucalyptus oil, ginger, licorice, copper gluconate (retsyn), thymol, and wintergreen, (2) citrus oils including lemon, orange, lime, grapefruit, yazu, sudachi, and (3) fruit essences including apple, pear, peach, grape, blueberry, strawberry, raspberry, cherry, plum, pineapple, banana, melon, apricot, ume, blackberry, tropical fruit, mango, mangosteen, pomegranate, papaya, watermelon, and so forth.

The flavoring agents can also provide a cooling sensation in the mouth. For example, useful cooling agents include menthol, xylitol, menthane, menthone, ketals, menthone ketals, menthone glycerol ketals, substituted p-menthanes, acyclic oarboxamides, substituted cyclohexanamides, substituted cyclohaxane carboxamides, substituted ureas and sulfonamides, substituted menthanols, hydroxymethyl and hydroxymethyl derivatives of p-menthane, 2-mercapto-cyclo-decanone, 2-isopropanyl-5- methylcyclohexanol, hydroxycarboxylic acids with 2-6 carbon atoms, cyclohexanamides, menthyl acetate, menthyl lactate, menthyl salicylate, N-2,3-trimethyl-2-isoptopyl butanamide (WS-23), N-ethyl-p-menmane-3-carboxamide (WS-3), menthyl succinate, 3,1-methoxypropane 1,2-diol, among others.

### D -NUTRACEUTICALS, HEALTH SUPPLEMENTS AND PROBIOTICS

The microparticles can also contain one or more nutraceuticals, health supplements and probiotics. Exemplary agents include, for example, but are not limited to, phytochemicals, glucosamine, methylsulfonylmethane, chondroitin, ruscus, bromlein, boswellin, carnitine, hydroxycitric acid, chitosan, acetyl-L-camitine, phosphatidylserine, huperzine-A, S-adenosylmethione, vinceptine, DMAE, lecithins, ginseng, ashwagandha, ipriflavone, NADH, magnesium malate, and D-ribose; minerals, such as, calcium, iodine, magnesium, zinc, iron, selenium, manganese, chromium, copper, cobalt, molybdenum, and phosphorus; vitamins, such as, vitamin C (ascorbic acid), vitamin A, vitamin B3, vitamin D (ergocalciferol), vitamin E (dl-alpha tocopherol), thiamine (vitamin B-1), riboflavin (vitamin B-2), niacin, pyridoxine (vitamin B-6), cyanocobalamin (vitamin B-12), folic acid, biotin, pantothenic Acid, and vitamin K; fatty acids, such as, the αmega-3 unsaturated fatty acids (gamma-linoleic acid, eicospentaenote acid, docosahexaenoic acid and the like); oils, such as, borage oil, high carotenoid canola oil, flax seed oil and mixtures thereof; nucleic acids, such as, DNA and RNA; essential amino acids, such as, tryptophan, lysine, methionine, phenylalanine, threonine, valine, leucine, and isoleucine; non-essential amino acids, such as, alanine, arginine, aspartic acid, cystine and cysteine, glutamic acid, glutamine, glycine, histidine, proline and hydroxyproline, serine, taurine, tyrosine and the like; enzymes, such as, bromelain, papain, amylase, cellulase, and coenzyme Q; microorganisms (for example, bacteria, such as, *Lactobacillus rhamnosus, Lactobacillus acidophilus, Lactobacillus bulgaricus, Lactobacillus bifldus, Loctobacillus plantarum,* and *Streptococcus faecium;* algae from sources, such as, *Spirulina* and *Chlorella*); antioxidants and phytochemicals, such as, anthocyanosides, carotenoids, bioflavonoids, glutathione, catechins, isoflavones, lycopene, ginsenosides, pycnogenol, alkaloids, pygeum phytosterols, sulforaphone, resveratrol, and grape seed extract and foods containing stanol esters, such as benecol; soy products; lecithin; and botanicals, such as herb extracts, and plant extracts.

### E - COSMETIC AGENTS

The microparticles can also contain one or more cosmetic agents. Exemplary cosmetic agents include, for example, moisturizers, emollients, fillers, colorants, perfumes or fragrances, skin conditioners and softeners (for example, urea), vitamins, photoprotectants (for example, sunscreens, such as p-dimethylaminobenzoic acid or glyceryl p-aminobenzoate), antiperspirants, antioxidants, anti-wrinkle materials, as well as any other materials suitable for topical applications; keratolytic agents, such as, salicylic acid; acne treating agents, such as, benzoyl peroxide or sulfur; perfumes, and the like, benzyl alcohol, disodium EDTA, hydroxylated lecithin, alkoxylated diester, polysorbate 80, polysorbate 65, and procaine hydrochloride.

### F -AGRICULTURAL AGENTS

The microparticles can also contain one or more agricultural agents. Exemplary agricultural agents include, for example, (1) pesticides, such as, growth regulators, photosynthesis inhibitors, pigment inhibitors, mitotic disrupters, lipid biosynthesis inhibitors, cell wall inhibitors, and cell membrane disrupters; (2) insecticides, such as, phosphoric esters such as azinphos-ethyl, azinphosmethyl, 1-(4-chlorophenyl)-4-(O- ethyl, S-propyl)-phosphoryloxypyrazole, chloropyrifos, coumaphos, demeton, demeton- S-methyl, diazinone, dichlorvos, dimethoate, ethoprophos, etrimfos, fenitrothion, penthion, heptenophos, parathion, paiathion-methyl, phosalone, phoxim, pirimiphos-ethyl, pirimiphos-methyl, piofenofos, protbiofbs, sulfprofos, triazophos and trichlorophone; (3) carbamates, such as, aldicarb, bendiocarb,2-(1-methylpropyl) phenyl methyl carbamate, butocarboxim, butoxycarboxim, carbaryl, carbofutan, carbosulfan, cloethocatb, isoprocarb, mefhomyl, oxamyl, pirimicarb, promecarb, propoxur and thiodicarb; (4) pyrethroids, such as, allethrin, alphamethrin, bioresmethrin, byfenthrin, oycloprothrin, cyfluthrin, decamethrin, cyhalothrin, cypermethrin, deltamethrin, alpha-cyano-3-phenyl-2-methylbenzyl 2,2-dimethyl-3-(2-chloro-2-trifluoro- methylvinyl)cyclopropanecarboxylate, fenpropathrin, fenfluthrin, fenvalerate, flucythrinate, flumethrin, fluvalinate, permethrin, resmethrin and tralomethrin; (S) nitroimines and nitromethylenes, such as, 1-[(6-chloro-3-pyridinyl)-methyl]-4,5-dihydro- N-nitro-1H-imidazol-2-amine- (imidacloprid); (6) herbicides, such as, alkanolaminc salts of dinitro-o-sec-butylphenol, propylene glycol butyl ethers of 2-(2,4,5-trichlorophenoxy) propanolic acid, chlorinated phenoxy acetic acid and salts or esters thereof, salts of 4- amino-3,5.6-dichloropicalinic acid; (7) fungicides, such as, 3a,4,7,7a-tetrahydro-2-[(trichloromethyl) thio]-1-H-isoindole-1,3 (2H)- dione, 3a,4,7,7a-tetrahydro-2-[(1,1,2,2-tetrachloro-ethyl)thio]-1-H-isoindole-1,3(2H)-dione, 2,4,S,6-tetrachloro-1,3-benzenedicarbonitrile, and sodium methyl- dithiocarbate; (8) agricultural petroleum distillates including light mineral oils and napthalates, and the like.

The amount of active agent or agents included in the microparticles will depend upon the intended use of the microparticles. It is understood that choice of the agent or agents and the amount of agent or agents included within the microparticles is within the level of skill in the art

It is understood that the microparticles of the invention can be used to deliver a microorganism, for example, bacteria, to a subject, The microorganism can still be viable. In certain embodiments, it is possible to adjust the conditions, for example, the spray drying conditions, so that at least 50%, 60%.70%, 80%, 90%, or 95% of the microorganisms remain viable after spray drying.

The invention will now be illustrated by means of the following examples which are given for the purpose of illustration only and without any intention to limit the scope of the present invention.

### EXAMPLES

### Example 1 - Microparticles Containing Tramadol or Caffeine

This example describes the synthesis and characterization of microparticles containing 16.5% (w/w) tramadol or 16.5% (w/w) caffeine.

The microparticles were produced as follows. CONTRAMID® excipient obtained from labopharm, Inc. (Laval, Canada) was dispersed in 0.2 M phosphate buffer pH 6.8-8 by high shear mixing (1800 rpm) using a MPS50 Turbo mixer (Robot@ Coupe, Jackson, MS) at 40-45°C for approximately 15 minutes to give a final dispersion having a concentration of 10% (w/w). KOLLIDON VA-64 Fine obtained from BASF (New Jersey) was solubilized in water, at room temperature, by vigorous stilling using a stirring bar for 10 minutes to give a final concentration of KOLLIDON VA-64 Fine of 20% (w/w). The active ingredient, for example, tramadol HCl (see TABLE 1) or caffeine (see TABLE 2) then was added to the aqueous solution of KOLLIDON VA-64 Fine to give the appropriate drug loading. The solution containing KOLLIDON VA-64 Fine and the active ingredient then was added gradually (over 20-23 minutes) to the solution containing the CONTRAMID excipient by high shear mixing (1800 rpm) using a MPS50 Turbo mixer (Robot@ Coupe, Jackson, MS) at room temperature. The resulting mixture was maintained under magnetic stilting over night ready for spray drying.

The resulting mixture then was spray dried in a mini Spray Dryer B-290 (BÜCHI Labortechnik AG, Switzerland) operating with co-current air and product stream. The air introduced via the air inlet had a temperature of 190°C. The outlet temperature and pressure were 85-90°C and 12 bar, respectively. The resulting microparticles were collected and characterized.

The composition of the solution spray dried to produce the tramadol-containing particles is described in TABLE 1, where the ratio of cross-linked high amylose starch (CONTRAMID): KOLLIDON VA-64 Fine was about 60:40.

**TABLE 1**

| Composition | Weight (g) |
|---|---|
| CONTRAMID | 46.8 |
| KOLLIDON VA-64 Fine | 31.3 |
| Tramadol HCl | 15.5 |
| 0.2 M Phosphate buffer | 420.8 |
| Water | 124.6 |

After spray drying, it is contemplated that the ratio of the cross-linked high amylose starch: KOLLIDON VA-64 Fine is about 60:40.

The composition of the solution spray dried to produce the caffeine-containing particles is described in TABLE 2, where the ratio of cross-linked high amylose starch (CONTRAMID): KOLLIDON VA-64 Fine was about 60:40.

**TABLE 2**

| Composition | Weight (g) |
|---|---|
| CONTRAMID | 46.8 |
| KOLLIDON VA-64 Fine | 31.3 |
| Caffeine | 15.5 |
| 0.2 M Phosphate buffer | 420.8 |
| Water | 124.6 |

After spray drying, it is contemplated that the ratio of the cross-linked high- amylose starch: KOLLIDON VA-64 Fine is about 60:40.

The resulting microparticles containing either tramadol or caffeine then were characterized by scanning electron microscopy (SEM) (JSM-840, JEOL, Tokyo, Japan) for microstructure characterization. Powder specimens of microspheres were fixed on the sample stubs with liquid adhesive, and then coated with a thin layer of gold using an ion sputter. The coated powder then was examined by SEM at an accelerating voltage of 15 kV. The SEM pictures were taken and visually analyzed for the microstructural properties of the spray-dried microencapsulated products.

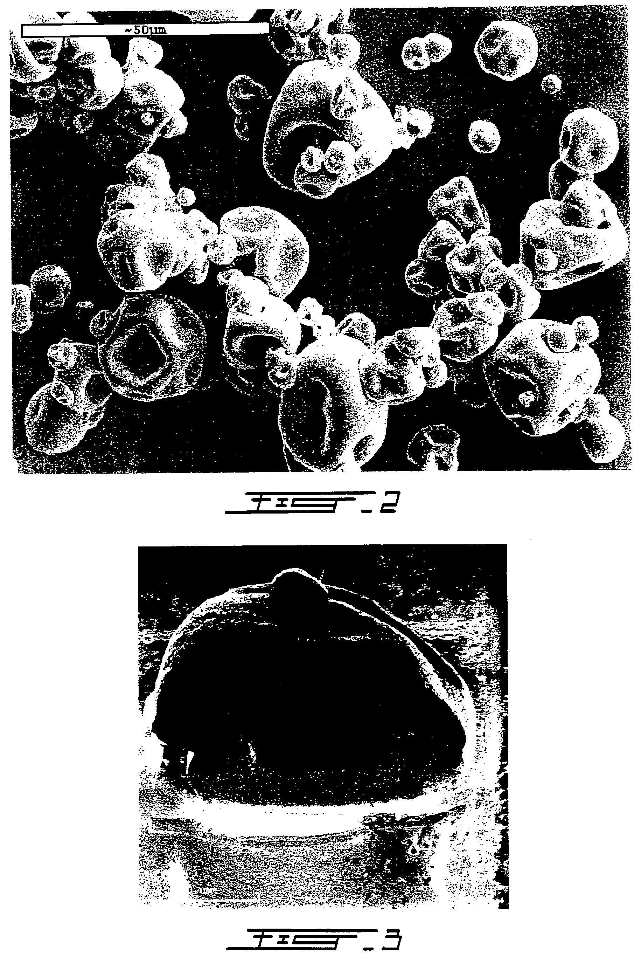

The resulting tramadol and caffeine containing microparticles had a mean diameter of about 1 µm to about 200 µm, with the majority (i.e., greater than 50%) of the particles having a mean diameter in the range of from about S µm to about 50 µm, An image of exemplary tramadol containing microparticles taken by SEM is shown in **FIGURE 2****.** A cross-sectional view of exemplary tramadol containing microparticles is shown in **FIGURE 3****,** which demonstrates that the microparticles were hollow.

The microparticles had a loading efficiency for both tramadol and caffeine of greater than 90 %. The final concentrations of the tramadol HCl and caffeine were about 16 % (w/w).

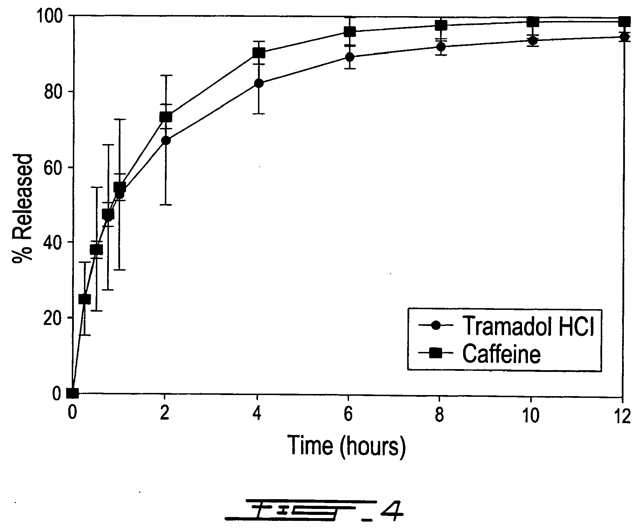

In addition, the release kinetics of the active agents from the microparticles were measured by a dissolution tester (VANKEL 10-1600, VK 700 from Varian) at a stirring speed of 100 rpm. Three 1.5 g samples from each batch of spray dried microparticles were tested using 900 mL of dissolution medium (water) maintained at 37°C. An aliquot of the release medium (S mL) was withdrawn at the following time intervals (0.2S, 0.5, 0.75, 1, 2, 4, 6, 8, 10 and 12 hours) and then analyzed. An equivalent amount of fresh dissolution medium, which was prewarmed at 37°C was replaced in the 900 mL dissolution bath. The collected samples then were analyzed for tramadol or caffeine content by HPLC. The results are summarized in **FIGURE 4****.**

**FIGURE 4** shows that both the tramadol and the caffeine were released with similar release kinetics with about 50-60% of the active ingredient being released by 2 hours, about 60-80% of the active ingredient being released by 3 hours, about 70-90% of the active ingredient being released by 4 hours, greater than about 80% of the active ingredient being released by 6 hours, and greater than about 90% of the active ingredient being released by 8 hours.

Drug release in the presence/absence of α-amylase was measured using the same dissolution tester (VANKEL 10-1600, VK 700) at a stirring speed of 100 rpm. Three 1,5 g samples of microparticles from each batch of spray dried microparticles were tested using 900 mL of dissolution medium (three baths with water and three other baths containing 5000 Units/L α-amylase) at 37°C. An aliquot of the release medium (5 mL) was withdrawn at the following time intervals (0,25, 0.5, 0.75, 1, 2, 4, 6, 8, 10 and 12 hours) and then analyzed. An equivalent amount of fresh dissolution medium, which was prewarmed at 37°C was replaced in the 900 mL dissolution bath. Collected samples then were analyzed for tramadol content by HPLC.

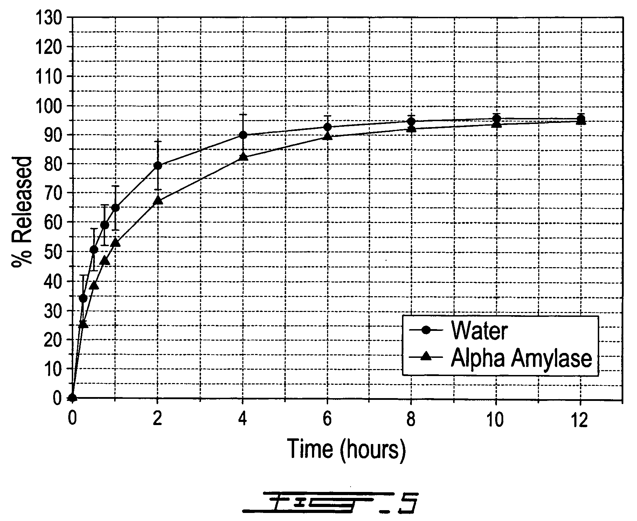

The results shown in **FIGURE 5** show that the microparticles of the invention are substantially resistant to α-amylase as the release profiles in both water and water containing α-amylase were substantially the same. The rate of tramadol release from the micropaiticles, if anything, was slightly slower in the presence of amylase versus than in the absence of amylase.

### Example 2 - Tramadol-Containing Microparticles with a High Loading Capacity

This example describes the synthesis and characterization of microparticles containing 50% (w/w) tramadol.

The microparticles containing tramadol were produced essentially as described in Example 1 except for the differing amounts of each of the initial ingredients that were combined prior to spray drying and the mixture also omitted KOLLIDON VA-64 Fine. The weight of the various starting materials are set forth in TABLE 3.

**TABLE 3**

| Composition | Weight (g) |
|---|---|
| CONTRAMID | 1,038.5 |
| Tramadol HCl | 1,038.5 |
| 0.2 M Phosphate buffer | 446.5 |
| Water | 15,303.5 |

CONTRAMID® excipient obtained from Labopharm, Inc. (Laval, Canada) was dispersed in phosphate buffer pH 6.8-8 by high shear mixing (1800rpm) vising the Robot Coupe MP 550 Turbo mixer at about 55°C for approximately 15 minutes to give a mixture containing 10 % (w/w) CONTRAMID® excipient. Tramadol then was added to the CONTRAMID® excipient-based dispersion under stirring. The mixture was stirred overnight at room temperature to allow for complete hydration.

On the following day, the CONTRAMID® excipient-based dispersion containing tramadol was heated to 55°C before being added to the spray dryer.

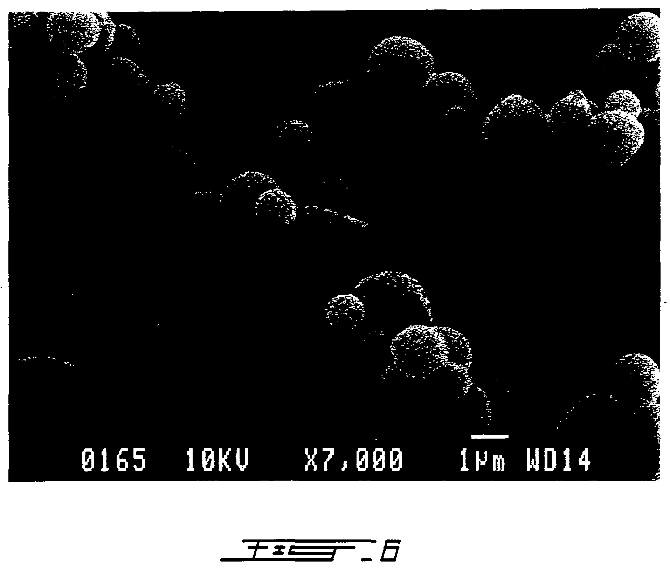

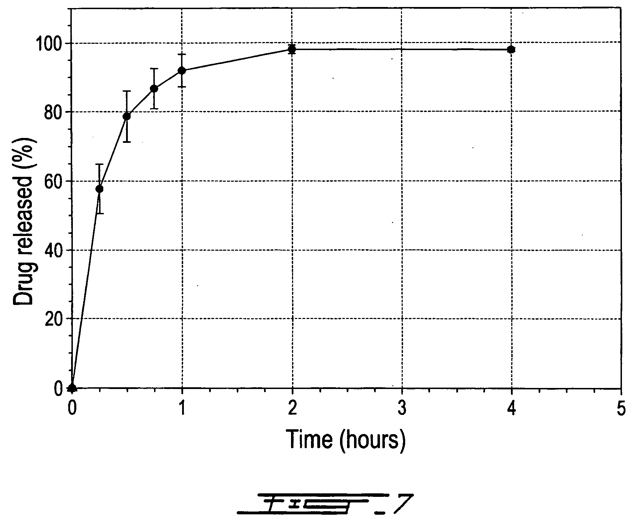

The resulting microparticles were characterized by SEM, the results of which are shown in FIGURE 6. Furthermore, the in vitro release kinetics were measured as described in Example 1 (the dissolution medium was amylase free water). The results are shown in FIGURE 7, wherein from about 70 % to about 90 % of the tramadol was released in 30 minutes, from about 80 % to about 95 % of the tramadol was released in 1 hour, and from about 90 % to about 100 % of the tramadol was released in 2 hours.

### Example 3 - Microparticles Conatining Agents with Low Melting Point

This example describes a method of incorporating a water insoluble agent with a low melting point, for example, menthol, into microparticles.

CONTRAMID® excipient from Labopkann, Inc. (Laval, Canada) was dispersed in phosphate buffer pH 6.8-8 by high shear mixing (1800 rpm) using a MP5S0 Turbo mixer (Robot® Coupe, Jackson, MS) at about 55°C for approximately 15 minutes to give a mixture containing 10% (w/w) CONTRAMID® excipient, The mixture was stirred overnight at room temperature to permit for complete hydration.

On the following day, the active agent (menthol, melting point about 42°C) was melted by heating to about 55°C and men was added to the CONTRAMID® excipient mixture and further mixed for 5 to 10 minutes using the Robot® Coupe MP550 Turbo mixer (12,000 rpm) (Robot® Coupe, Jackson, MS) to form a milky white emulsion, Optionally, a surface active agent, such as, Tween® 20 or Tween® 80, could be added to improve the stability of the emulsion. The emulsion then was continuously mixed at about 50°C to 70°C before being dried by a spray dryer, as described in Example 1.

### Example 4 - Microparticles Containing Agents with High Melting Point

This example describes the method of incorporating a water insoluble agent with a high melting point, such as vanillin, into microparticles.

CONTRAMID® excipient obtained from Labopharm, Inc. (Laval, Canada) was dispersed in phosphate buffer pH 6.8-8 by high shear mixing (1800 rpm) using a MP550 Turbo mixer (Robot@ Coupe, Jackson, MS) at about 55°C for approximately 15 minutes to give a mixture containing 10 % (w/w) CONTRAMID® excipient The mixture was stirred overnight at room temperature to allow for complete hydration.

On the following day, the active agent (vanillin, melting point about 85°C) was dissolved in a suitable dispersing agent (namely, mineral oil or castor oil), and heated to 55°C. The oily solution then was added to the CONTRAMID® excipient mixture by high shear mixing (12,000 rpm) using a MP550 turbo mixer (Robot® Coupe, Jackson, MS) to form a milky white emulsion. Optionally, a surface active agent, such as, Tween® 20 or Tween® 80, could be added to the emulsion in order to improve the stability of the emulsion. The emulsion was continuously mixed at about 70°C before being spray dried, as described in Example 1.

### Example 5 - Contramid® Microparticlcs Containing a Probiotic Agent

This example describes the preparation and characterization of Contramid® excipient-based microparticles containing a probiotic agent, *Lactobacillus rhamnosus* HA-I 11, at concentration level of 8.5 x 10⁹ cfu/mL.

*Lactobacillus rhamnosus* is a probiotic bacterium that can slow or inhibit the growth of harmful bacteria in the intestine. It is often used as a natural preservative in yogurt and other dairy products to extend shelf life.

Microparticles containing the probiotic agent were produced essentially as described in Example 1. The CONTRAMID®excipient was first dispersed in phosphate buffer pH 6.8-8 by high shear mixing (1800 rpm) using a MPS50 Turbo mixer (Robot^{®} Coupe, Jackson, MS) at 40-45°C for approximately 15 minutes to give a final dispersion having a concentration of 10% (w/w). KOLLIDON VA-64 Fine obtained from BASF (New Jersey) was solubilised in water, at room temperature, by vigorous stirring for 10 minutes to give a final concentration of KOLLIDON VA-64 Fine of 20% (w/w). The KOLLIDON VA-64 solution then was mixed with the CONTRAMID<16> dispersion and the dispersion then was cooled to a temperature in the range of 6°C - 12°C by circulating cold water (4°C) through the jacket of the container. The probiotic suspension then was added to the CONTRAMÏD® based dispersion under low agitation. The weight of the various starting materials is set forth in TABLE 4.

**TABLE 4**

| Composition | Weight (g) |
|---|---|
| CONTRAMID^{®} | 960 |
| KOLLIDON VA-64 | 400 |
| *Lactobacillus rhamnosus* HA-111 suspension(8.5x10⁹cfu/mL) | 9000 |
| 0.2 M Phosphate buffer | 347 |
| Water | 11893 |

The dispersion was continuously mixed at about 7°C before spray drying, as described in Example 1. The air inlet temperature was 180°C, and the air outlet temperature was either 60°C or 80°C.

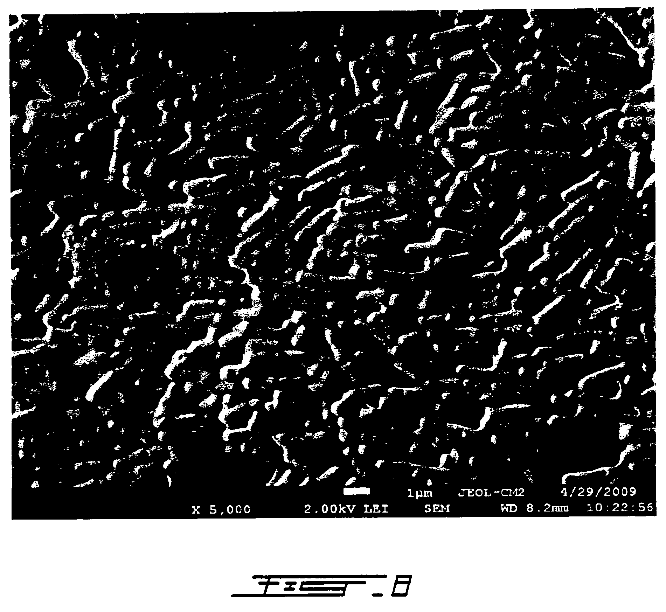

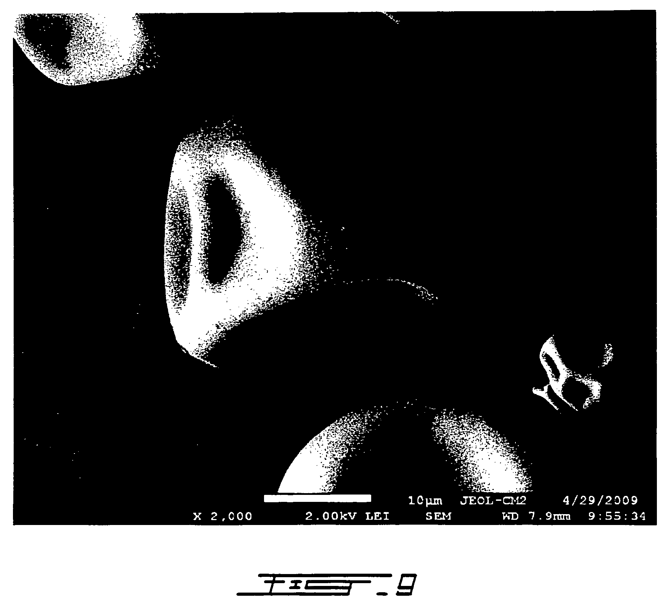

An image of the bacterial strain *Lactobacillus rhamnosus* taken by SEM is shown in **FIGURE 8****,** and an image of the resulting spray dried microparticles also taken by SEM is shown in **FIGURE 9****.** It is believed that the bacteria were encapsulated into CONTRAMID® excipient-based microparticles after spray drying in view of the absence of rods in **FIGURE 9****.**

The yield (colony forming units (cfu)) of surviving bacteria present in the microparticles after spray drying is summarized in Table 5.

**TABLE 5**

| Air outlet temperature (°C) | Initial cfu before spray drying | cfu after spray drying | bacterial survival (%) after drying |
|---|---|---|---|
| 60°C | 8.5x10⁹ | 8.5x10⁹ | ≥95 |
| 80°C | 8.5x10⁹ | 2x10⁹ | 23.5 |

From TABLE 5, the survival rate of *Lactobacillus rhamnosus* after spray drying was greater than 95 % at 60°C and about 23,5% at 80°C.

It is contemplated that other bacterial strains can be introduced into the microparticles according to the invention, for example, *Lactobacillus acidophilus. Lactobacillus bulgaricus, Lactobacillus plantarum, Stqphococcusfaecium,* and the like.

### Example 6 - CONTRAMID® Microparticles Containing a Herbal Products

This example describes the preparation and characterization of CONTRAMID® excipient-based microparticles containing a herbal product.

The microparticles containing a blend of plant extracts were produced essentially as described in Example 1. The CONTRAMID® excipient was first dispersed in 0.2 M phosphate buffer pH 6.8-8 by high shear mixing (1800 rpm) using a MPS50 Turbo mixer (Robot@ Coupe, Jackson, MS) at 40-45°C for approximately 15 minutes to give a final dispersion having a concentration of 10 % (w/w). A blend of herbal extracts was suspended in 0.2 M phosphate buffer, pH 6.8 containing 0.25% (w/v) soy lecithin and maintained under mild agitation overnight to avoid sedimentation of particles. The herbal blend suspension then was added to the CONTRAMID® dispersion under agitation.

The weight of the various starting materials is set forth in TABLE 6.

**TABLE 6**

| Composition | Weight (g) |
|---|---|
| CONTRAMID^{®} | 750 |
| Soy lecithin | 10 |
| Herbal blend | 750 |
| 0.2 M Phosphate buffer | 333 |
| Water | 11417 |

The dispersion was continuously mixed at room temperature before being spray dried, as described in Example 1. The air inlet temperature was 190°C, and the air outlet temperature was 70°C.

The resulting spray dried microparticles resulted in a fine powder that was readily dispersible in water as opposed to initial herbal blend that was not readily dispersible in water. The microparticles also resulted in significant taste and odor masking of the initial herbal blend making it more palatable for consumption.

## Claims

1. A method of producing microparticles that stabilize and/or control the release of an agent disposed therein, the method comprising
(a) providing a solution comprising a mixture of from 5 % w/w to 20 % w/w of cross-linked high amylose starch and the agent to be released; and
(b) spray drying the mixture of step (a) in a spray dryer to produce microparticles having a mean diameter of from 1 µm to 200 µm.

2. The method of claim 1, wherein the microparticles have a mean diameter in the range from 5 µm to 200 µm, from 5 µm to 150 µm, from 1 µm to 100 µm m, from 10 µm to 100 µm, from 1 µm to 50 µm, from 1 µm to 40 µm, from 1 µm to 30 µm, from 2 µm to 50 µm, from 3 µm to 50 µm, from 4 µm to 50 µm, or from 5 µm to 50 µm.

3. The method of claim 1 or 2, wherein the mixture provided in step (a) comprises from 7 % w/w to 15% w/w of cross-linked high amylose starch.

4. The method of any one of claims 1-3, wherein the agent comprises from 5 % to 50 % (w/w) of the microparticles produced in step (b).

5. The method of claim 4, wherein the agent to be released comprises from 10 % to 45 % (w/w), from 15 % to 40 % (w/w), from 15 % to 35 % (w/w) or from 20 % to 35 % (w/w) of the microparticles produced in step (b).

6. The method of any one of claims 1 -5, wherein the mixture provided in step (a) is created by mixing the solution comprising the cross-linked high amylose starch and the agent at a temperature in the range of from 15 °C to 70 °C.

7. The method of claim 6, wherein the temperature is in the range of from 30 °C to 60 °C.

8. The method of any one of claims 1-7, wherein, in step (a), the agent is melted prior to mixing with the cross-linked high amylose starch.

9. The method of any one of claims 1-8, wherein, in step (a), the agent is mixed with a dispersing agent prior to mixing with the cross-linked high amylose starch.

10. The method of claim 8 or 9, wherein, in step (a), the solution further comprises a surface active agent.

11. The method of claim 10, wherein the surface active agent is selected from the group consisting of fatty acids and salts, glycerides and esters thereof, sorbitan esters, polysorbates, polyoxyethylene stearates, poloxamers, and sodium dodecyl sulphate.

12. The method of any one of claims 1-11, wherein, in step (a), the solution further comprises a viscosity reducing agent.

13. The method of claim 12, wherein the viscosity reducing agent is selected from the group consisting of a water soluble linear polymer, a water soluble linear copolymer, and a polyol.

14. The method of claim 12, wherein the viscosity reducing agent is selected from the group consisting of a polyvinylpyrrolidone-vinyl acetate copolymer, polyvinyl pyrrolidone, propylene glycol, sorbitol, and mannitol.

15. The method of claim 12, wherein the viscosity reducing agent is a polyvinylpyrrolidone-vinyl acetate copolymer.

16. A microparticle composition comprising:
a plurality of microparticles, produced according to a method of any of claims 1 to 15,
comprising from 35 % (w/w) to 70 % (w/w) of cross-linked high amylose starch, an agent for release from the microparticles, and a viscosity reducing agent wherein the microparticles have a mean diameter in the range from 1 µm to 200 µm.

17. The composition of claim 16, wherein the microparticles have a mean diameter in the range from 5 µm to 200 µm, from 5 µm to 150 µm, from 1 µm to 100 µm m, from 10 µm to 100 µm, from 1 µm to 50 µm, from 1 µm to 40 µm, from 1 µm to 30 µm, from 2 µm to 50 µm, from 3 µm to 50 µm, from 4 µm to 50 µm, or from 5 µm to 50 µm.

18. The composition of claim 16, wherein the viscosity reducing agent is selected from the group consisting of a water soluble linear polymer, a water soluble linear copolymer, and a polyol,

19. The composition of claim 16, wherein the viscosity reducing agent is selected from the group consisting of a copolymer of vinyl acetate and vinyl pyrrolidone, polyvinyl pyrrolidone, propylene glycol, sorbitol, and mannitol.

20. The composition of any one of claims 16-19, wherein the microparticles are substantially free of pectin.

21. The composition of any one of claims 16-20, wherein the microparticles are substantially resistant to degradation by α-amylase.

22. The composition of any one of claims 16-21, wherein the agent comprises from 5 % to 50 % (w/w) of the microparticles.

23. The composition of claim 22, wherein the agent comprises from 10 % to 45 % (w/w), from 15 % to 40 % (w/w), from 15 % to 35 % (w/w), or from 20 % to 35 % (w/w) of the microparticles.

## Patentansprüche

1. Verfahren zur Herstellung von Mikropartikeln, die ein darin befindliches Agens stabilisieren und/ oder dessen Freisetzung kontrollieren, das Verfahren umfassend
(a) Bereitstellen einer Lösung umfassend eine Mischung von 5 % Gew./Gew. bis 20 % Gew./Gew. von vernetzter amylosereicher Stärke und das freizusetzende Agens; und
(b) Sprühtrocknen der Mischung aus Schritt (a) in einem Sprühtrockner, um Mikropartikel mit einem mittleren Durchmesser von 1 µm bis 200 µm herzustellen.

2. Verfahren nach Anspruch 1, wobei die Mikropartikel einen mittleren Durchmesser im Bereich von 5 µm bis 200 µm, von 5 µm bis 150 µm, von 1 µm bis 100 µm, von 10 µm bis 100 µm, von 1 µm bis 50 µm, von 1 µm bis 40 µm, von 1 µm bis 30 µm, von 2 µm bis 50 µm, von 3 µm bis 50 µm, von 4 µm bis 50 µm, oder von 5 µm bis 50 µm haben.

3. Verfahren nach Anspruch 1 oder 2, wobei die in Schritt (a) bereitgestellte Mischung von 7 % Gew./Gew. bis 15% Gew./Gew. vernetzter amylosereicher Stärke umfasst.

4. Verfahren nach einem der Ansprüche 1-3, wobei das Agens von 5 % bis 50 % (Gew./Gew.) der in Schritt (b) hergestellten Mikropartikel umfasst.

5. Verfahren nach Anspruch 4, wobei das freizusetzende Agens von 10 % bis 45 % (Gew./Gew.), von 15 % bis 40 % (Gew./Gew.), von 15 % bis 35 % (Gew./Gew.) oder von 20 % bis 35 % (Gew./Gew.) der in Schritt (b) hergestellten Mikropartikel umfasst.

6. Verfahren nach einem der Ansprüche 1-5, wobei die in Schritt (a) bereitgestellte Mischung durch Mischen der Lösung umfassend die vernetzte amylosereiche Stärke und des Agens bei einer Temperatur im Bereich von 15 °C bis 70 °C erzeugt wird.

7. Verfahren nach Anspruch 6, wobei die Temperatur in einem Bereich von 30 °C bis 60 °C liegt.

8. Verfahren nach einem der Ansprüche 1-7, wobei, in Schritt (a), das Agens vor dem Mischen mit der vernetzten amylosereichen Stärke geschmolzen wird.

9. Verfahren nach einem der Ansprüche 1-8, wobei, in Schritt (a), das Agens vor dem Mischen mit der vernetzten amylosereichen Stärke mit einer dispergierenden Substanz gemischt wird.

10. Verfahren nach Anspruch 8 oder 9, wobei, in Schritt (a), die Lösung weiterhin ein oberflächenaktives Agens umfasst.

11. Verfahren nach Anspruch 10, wobei das oberflächenaktive Agens ausgewählt ist aus der Gruppe bestehend aus Fettsäuren und Salzen, Glyceriden und Estern davon, Sorbitanestern, Polysorbaten, Polyoxyethylenstearaten, Poloxameren, und Natriumdodecylsulfat.

12. Verfahren nach einem der Ansprüche 1-11, wobei, in Schritt (a), die Lösung weiterhin ein viskositätsverringerndes Agens umfasst.

13. Verfahren nach Anspruch 12, wobei das viskositätsverringernde Agens ausgewählt ist aus der Gruppe bestehend aus einem wasserlöslichen, linearen Polymer, einem wasserlöslichen, linearen Copolymer, und einem Polyol.

14. Verfahren nach Anspruch 12, wobei das viskositätsverringernde Agens ausgewählt ist aus der Gruppe bestehend aus einem Polyvinylpyrrolidon-Vinylacetat-Copolymer, Polyvinylpyrrolidon, Propylenglykol, Sorbitol, und Mannitol.

15. Verfahren nach Anspruch 12, wobei das viskositätsverringernde Agens ein Polyvinylpyrrolidon-Vinylacetat- Copolymer ist.

16. Mikropartikelzusammensetzung umfassend:
eine Vielzahl von Mikropartikeln, hergestellt nach einem Verfahren gemäß einem der Ansprüche 1 bis 15, umfassend von 35 % (Gew./Gew.) bis 70 % (Gew./Gew.) vernetzter amylosereicher Stärke, ein Agens zur Freisetzung von den Mikropartikeln, und ein viskositätsverringerndes Agens, wobei die Mikropartikel einen mittleren Durchmesser im Bereich von 1 µm bis 200 µm haben.

17. Zusammensetzung nach Anspruch 16, wobei die Mikropartikel einen mittleren Durchmesser im Bereich von 5 µm bis 200 µm, von 5 µm bis 150 µm, von 1 µm bis 100 µm, von 10 µm bis 100 µm, von 1 µm bis 50 µm, von 1 µm bis 40 µm, von 1 µm bis 30 µm, von 2 µm bis 50 µm, von 3 µm bis 50 µm, von 4 µm bis 50 µm, oder von 5 µm bis 50 µm haben.

18. Zusammensetzung nach Anspruch 16, wobei das viskositätsverringernde Agens ausgewählt ist aus der Gruppe bestehend aus einem wasserlöslichen, linearen Polymer, einem wasserlöslichen, linearen Copolymer, und einem Polyol.

19. Zusammensetzung nach Anspruch 16, wobei das viskositätsverringernde Agens ausgewählt ist aus der Gruppe bestehend aus einem Copolymer von Vinylacetat und Vinylpyrrolidon, Polyvinylpyrrolidon, Propylenglykol, Sorbitol, und Mannitol.

20. Zusammensetzung nach einem der Ansprüche 16-19, wobei die Mikropartikel im Wesentlichen frei von Pektin sind.

21. Zusammensetzung nach einem der Ansprüche 16-20, wobei die Mikropartikel im Wesentlichen beständig gegen Degradation durch α-Amylase sind.

22. Zusammensetzung nach einem der Ansprüche 16-21, wobei der Agens von 5 % bis 50 % (Gew./Gew.) der Mikropartikel umfasst.

23. Zusammensetzung nach Anspruch 22, wobei der Agens von 10 % bis 45 % (Gew./Gew.), von 15 % bis 40 % (Gew./Gew.), von 15 % bis 35 % (Gew./Gew.), oder von 20 % bis 35 % (Gew./Gew.) der Mikropartikel umfasst.

## Revendications

1. Procédé de production de microparticules qui stabilisent et/ou régulent la libération d'un agent placé dedans, lequel procédé comporte les étapes suivantes :
a) préparer une solution comprenant un mélange de 5 % en poids à 20 % en poids d'amidon réticulé à haute teneur en amylose et de l'agent à libérer ;
b) et faire sécher par atomisation le mélange obtenu dans l'étape (a), dans un appareil de séchage par atomisation, de manière à produire des microparticules dont le diamètre moyen vaut de 1 µm à 200 µm.

2. Procédé conforme à la revendication 1, dans lequel les microparticules présentent un diamètre moyen situé dans l'intervalle allant de 5 µm à 200 µm, de 5 µm à 150 µm, de 1 µm à 100 µm, de 10 µm à 100 µm, de 1 µm à 50 µm, de 1 µm à 40 µm, de 1 µm à 30 µm, de 2 µm à 50 µm, de 3 µm à 50 µm, de 4 µm à 50 µm, ou de 5 µm à 50 µm.

3. Procédé conforme à la revendication 1 ou 2, dans lequel le mélange préparé dans l'étape (a) comprend de 7 % en poids à 15 % en poids d'amidon réticulé à haute teneur en amylose.

4. Procédé conforme à l'une des revendications 1 à 3, dans lequel l'agent représente de 5 % à 50 % en poids des microparticules préparées dans l'étape (b).

5. Procédé conforme à la revendication 4, dans lequel l'agent à libérer comprend de 10 % à 45 % en poids, de 15 % à 40 % en poids, de 15 % à 35 % en poids, ou de 20 % à 35 % en poids des microparticules préparées dans l'étape (b).

6. Procédé conforme à l'une des revendications 1 à 5, dans lequel, dans l'étape (a), on prépare le mélange en mélangeant l'agent et la solution comprenant l'amidon réticulé à haute teneur en amylose à une température située dans l'intervalle allant de 15 °C à 70 °C.

7. Procédé conforme à la revendication 6, dans lequel la température est située dans l'intervalle allant de 30 °C à 60 °C.

8. Procédé conforme à l'une des revendications 1 à 7, dans lequel, dans l'étape (a), on fait fondre l'agent avant de le mélanger avec l'amidon réticulé à haute teneur en amylose.

9. Procédé conforme à l'une des revendications 1 à 8, dans lequel, dans l'étape (a), on mélange l'agent avec un agent dispersant avant de le mélanger avec l'amidon réticulé à haute teneur en amylose.

10. Procédé conforme à la revendication 8 ou 9, dans lequel, dans l'étape (a), la solution comprend en outre un agent tensioactif.

11. Procédé conforme à la revendication 10, dans lequel l'agent tensioactif est choisi dans l'ensemble constitué par les acides gras et leurs sels, esters et glycérides, les esters de sorbitanne, les polysorbates, les stéarates de poly(oxyéthylène), les poloxamères et le dodécyl-sulfate de sodium.

12. Procédé conforme à l'une des revendications 1 à 11, dans lequel, dans l'étape (a), la solution comprend en outre un agent de réduction de la viscosité.

13. Procédé conforme à la revendication 12, dans lequel l'agent de réduction de la viscosité est choisi dans l'ensemble constitué par un polymère linéaire hydrosoluble, un copolymère linéaire hydrosoluble et un polyol.

14. Procédé conforme à la revendication 12, dans lequel l'agent de réduction de la viscosité est choisi dans l'ensemble constitué par un copolymère poly(vinyl-pyrrolidone/acétate de vinyle), une poly(vinyl-pyrrolidone), le propylèneglycol, le sorbitol et le mannitol.

15. Procédé conforme à la revendication 12, dans lequel l'agent de réduction de la viscosité est un copolymère poly(vinyl-pyrrolidone-/acétate de vinyle).

16. Composition de microparticules, comprenant un ensemble de multiples microparticules produites selon un procédé conforme à l'une des revendications 1 à 15, comprenant de 35 % en poids à 70 % en poids d'amidon réticulé à haute teneur en amylose, un agent à libérer à partir des microparticules et un agent de réduction de la viscosité, dans laquelle les microparticules présentent un diamètre moyen situé dans l'intervalle allant de 1 à 200 µm.

17. Composition conforme à la revendication 16, dans laquelle les microparticules présentent un diamètre moyen situé dans l'intervalle allant de 5 µm à 200 µm, de 5 µm à 150 µm, de 1 µm à 100 µm, de 10 µm à 100 µm, de 1 µm à 50 µm, de 1 µm à 40 µm, de 1 µm à 30 µm, de 2 µm à 50 µm, de 3 µm à 50 µm, de 4 µm à 50 µm, ou de 5 µm à 50 µm.

18. Composition conforme à la revendication 16, dans laquelle l'agent de réduction de la viscosité est choisi dans l'ensemble constitué par un polymère linéaire hydrosoluble, un copolymère linéaire hydrosoluble et un polyol.

19. Composition conforme à la revendication 16, dans laquelle l'agent de réduction de la viscosité est choisi dans l'ensemble constitué par un copolymère d'acétate de vinyle et de vinyl-pyrrolidone, une poly(vinyl-pyrrolidone), le propylèneglycol, le sorbitol et le mannitol.

20. Composition conforme à l'une des revendications 16 à 19, dans laquelle les microparticules ne contiennent pratiquement pas de pectine.

21. Composition conforme à l'une des revendications 16 à 20, dans laquelle les microparticules résistent bien à une dégradation causée par une α-amylase.

22. Composition conforme à l'une des revendications 16 à 21, dans laquelle l'agent comprend de 5 % à 50 % en poids des microparticules.

23. Composition conforme à la revendication 22, dans laquelle l'agent comprend de 10 % à 45 % en poids, de 15 % à 40 % en poids, de 15 % à 35 % en poids, ou de 20 % à 35 % en poids des microparticules.
